# EUROPEAN PATENT SPECIFICATION

(11) **EP 0 507 839 B1**
(45) Date of publication and mention of the grant of the patent: **05.04.1995**
(21) Application number: 91902106.3
(22) Date of filing: 28.12.1990
(51) Int. Cl.: C12N 5/08, A61K 35/28, C12P 21/08

(54) **METHOD OF SEPARATING HAEMOPOIETIC PROGENITOR CELLS**
TRENNUNGSVERFAHREN ZUM TRENNEN VON HÄMATOPOEITISCHEN VORLÄUFERZELLEN
PROCEDE DE SEPARATION DE CELLULES PARENTES HEMATOPOIETIQUES

(30) Priority: 29.12.1989 GB 8929297
(43) Date of publication of application: 14.10.1992
(73) Proprietor: DYNAL AS, 0212 Oslo 2 (NO)
(72) Inventor: FUNDERUD, Steinar, N-0756 Oslo 7 (NO); SMELAND, Erland, Bremerthun, N-0286 Oslo 8 (NO)
(74) Representative: Holmes, Michael John
(86) International application number: EP9002327
(87) International publication number: WO9109938

(56) References cited:
- LEUKEMIA, vol. 2, no. 10, 1988, Oliver G. Ottmann et al.: see page 677 - page 686
- CANCER RESEARCH, vol. 47, 1987, Gunnar Kvalheim et al.: see page 846 - page 851
- J. Clin. Invest., vol. 81, 1988, Ronald J. Berenson et al.: see page 951 - page 955
- EXP. HEMATOL., vol. 14, 1986, J.H. Frederik, Falkenburg et al.: see page 101 - page 107
- PROGRESS IN CLINICAL AND BIOLOGICAL RESEARCH, vol. 333, 1990, Curt I. Civin et al.: see page 387 - page 402, see especially pages 389-390
- Dialog Information Services, File 155, MEDLINE 66-91/March, MEDLINE Accession no. 89001406, JT Kemshead et al.: & Bone Marrow Transplant Aug 1987, 2 (2) p 133-9, abstract

## Description

This invention relates to an immunomagnetic method of selecting haemopoietic progenitor cells (HPC) from bone marrow and other mixed cell populations.

Autologous bone marrow transplantation after supralethal treatment by high dose chemotherapy and/or total irradiation is used in treating tumour forms such as leukemias, lymphomas, small cell lung cancer and neuroblastomas. It is important for success in such transplantation that the autologous bone marrow is free from malignant cells. Methods which have been attempted for ex vivo purging of bone marrow include non specific treatment with cytotoxic agents or specific treatment using tumour associated monoclonal antibodies. The method most extensively used so far for removal of B-lymphoma cells from human bone marrow involves the use of monoclonal antibodies plus complement. Using a mixture of 3 different monoclonal antibodies and complement and 3 cycles of treatment, Bast et al. (Cancer Res. 45, 499-503,1985) have obtained up to 5 logs of depletion of Burkitt tumour cells admixed with irradiated human bone marrow. However, the procedure has several shortcomings. It requires several washings with consequent cell loss and is rather time consuming. Moreover, complement is difficult to standardize. Another difficulty is that normal bone marrow seems to produce an anticomplementary factor. Thus high concentrations of bone marrow cells inhibit the effect of complement.

Extensive studies on bone marrow purging using magnetic particles have to our knowledge, only been reported in neuroblastoma wherein the bone marrow is first incubated with a cocktail of antineuroblastoma antibodies and subsequently with magnetic beads covered with anti-antibodies.

The problem with such purging techniques is the heterogenicity of tumour cell populations and the existence of tumour cells which express little or no surface antigen. It has thus been proposed to use several antitumour antibodies to remove a number of different tumour cell types but it is virtually impossible to remove all tumour cells in this way.

An alternative approach is to select positively the desired bone marrow cells thus leaving the malignant cells behind. However, it has proved difficult to find a surface antigen which is reliably present on the desired haemopoietic cells but not on malignant cells derived from the haemopoietic cell population, for example lymphoma cells.

Thus, the antigen CD 34 is expressed by HPC. In the first stage of differentiation into colony forming cells (e.g. CFU-GEMM) these express antigens CD33 and CD34. In the next stage of differentiation to cells of the erythroid, myelomonocytic and megakaryotic lineages, the vital BFU_{E} cells of the erythroid lineage carry antigens CD33 and CD 34 although these are lost in later differentiation. The myelomonocytic lineage includes CFU GM cells which carry CD33 but not CD34 although CD33 is subsequently lost. The megakaryotic lineage leads initially to CFU Mega cells which carry CD34 which is subsequently lost. Thus, monoclonal antibodies against antigen CD34 (and possibly CD33) provide one suitable method for selecting early haemopoietic cells which theoretically should be less likely to be malignant although using a column separation technique, only limited enrichment has been achieved (Berenson R J et al; J Clin. Invest. 1988, 81, 951-960). Unfortunately, there are a few malignant cells found in acute leukaemia and some chronic myelogenous leukaemia blasts which have been found to express CD34 and CD33 so that this approach is not completely reliable.

A further significant system of antigens on HPC and other cells is the MHC (major histocompatibility complex) Class II group. It has been found that the majority of HPC carry an antigen termed DR and on differentiation express an antigen termed DP and then a further antigen termed DQ. Thus, the MHC Class II DR antigen is characteristic of relatively early stem cells. We have described (Cancer Research 47, 846-851, 1987) a novel monoclonal antibody AB-4 which is active against cells carrying DR antigen but not against all HPC. The DR epitopes recognized by AB-4 clearly have a more restricted expression on HPC compared with the monomorphic DR epitopes recognised by most other antibodies against monomorphic DR antigens. AB-4 is thus capable when bound to an inert support, of removing from a total population of haemopoietic cells, the greater number of the more mature cells including, in particular, B cells and any leukaemia cells while leaving a fraction of HPC in the supernatant. We have now found that positive selection of HPC, before or after elimination of AB-4 cells, using a monoclonal antibody specific for an antigen on the stem cells, e.g. CD34 antigen, provides a method of safely eliminating leukaemia cells from the stem cell population. A similar safe system may also be achieved by replacing AB-4 by a monomorphic DP or DQ specific antibody.

According to the present invention therefore we provide a method of separating haemopoietic progenitor cells (HPC) from within a mixed population of haemopoietic cells, which may contain malignant cells, characterised in that (1) said mixed population is treated with one or more negative selection antibodies reactive with the DP, DQ or DR antigens of the MHC Class II, but not with the monomorphic epitope of the DR antigen on HPC, said negative selection antibody(s) being bound to magnetic particles before or after binding to said cells and the magnetic particles and attached negative selection antibody and cells being removed to leave a negatively selected population of cells including at least HPC, and (2) HPC and any cells associated therewith are treated with a positive selection antibody reactive with an antigen on said HPC, said antibody being bound to magnetic particles before or after binding to cells carrying said antigen, the magnetic particles and attached cells being separated from other cells by magnetic aggregation and the cells optionally being liberated from the magnetic particles to leave a positively selected population of cells containing said HPC, steps (1) and (2) being effected in either order so that the said positively selected population of cells is the mixed population treated in step (1) or the said negatively selected population of cells are the cells treated in step (2).

The preferred negative selection antibody or antibody mixture is one which is reactive with DR, DP and DQ antigens of the MHC Class II other than the monomorphic epitope of the DR antigen on HPC, especially monoclonal antibody AB-4 referred to above. Monoclonal antibody FN81.1 which recognises the DQ antigen and 22C1 which recognises the DP antigen may also be used. These antibodies may optionally be used together with a B-cell specific antibody such as monoclonal antibody AB-1 (which recognises CD19 antigen), also described in the same publication (Cancer Research 47, 846-851, 1987) and/or an antibody against T cells such as anti-CD2 or anti-CD7 or one or more antibodies against myeloid cells such as anti-CD33, anti-CD15 or anti-CD36. Antibodies AB-4 and AB-1 are both IgM and in general IgM antibodies are preferred to IgG antibodies, partly on the basis of their ease of liberation from the cells after positive selection as described hereinafter. Both the above monoclonal antibodies were obtained from hydridomas between X63 Ag 8.653 cells and spleen cells taken from a BALB/c mouse immunised with cells taken from a patient with diffuse centroblastic B-cell lymphoma. AB-4 has been shown to recognise a monomorphic DR W52 antigen; clearly this antigen is not a monomorphic DR antigen expressed on stem cells, at least in a form capable of binding to antibody AB-4. The selected sub population of cells obtained by positive selection with anti-CD34 magnetic beads has been successfully grown to produce blast cells. It appears that the particular mixture of naive pluripotent cells and some cells carrying the monomorphic DR antigen but not the DP and DQ antigens may be beneficial in securing blast cell growth. It is notable that pluripotent haemopoietic stem cells alone have failed to engraft in lethally irradiated mice (Jones etal, Nature, 347, 13 Sept 1990).

The positive selection antibody may, for example, be an antibody reactive with the CD34 antigen or another broadly expressed HPC antigen. More broadly active antibodies are also of value since the negative selection step will remove unwanted cells included within the wider antigen groupings and leave only the desired HPC. Thus, it is possible to use, for example, HKB1 which is a pan class II specific (Holte, H. et al. Eur.J.Immunnol. 19, 1221-1225: 1989) IgM antibody. A further candidate for positive selection is an antibody AB-3 (IgG) which also recognises a monomorphic DR antigen on stem cells (Holte, H. et al. Eur.J.Immunnol. 19, 1221-1225: 1989).

It was not previously known whether or not all of the HPC remaining after negative selection with AB-4, carried antigens against which antibodies were available. However, it has been found that anti-CD34 binds to a population of HPC not recognised by the so-called pan MHC Class II specific antibody HKB1, and which are at an earlier stage of differentiation. This population comprises pluripotent HPC, which are particularly suitable for bone marrow replacement.

Consequently, anti-CD34 is preferred as a positive-selection antibody and anti HKB1 is less preferred since is will not recognise an important sub-population of the HPC. Useful anti-CD34 antibodies include B1-3C5 (Tindle WR et al. Leukemia Research 1985, 9:1-9) and 12.8 (Andrews RG et al, Blood 67:842-45 (1986)).

The monoclonal antibodies used for both positive and negative selection may be attached to the magnetic particles in a number of ways. The magnetic particles may be coated with a substance capable of binding to the antibodies reversibly without hindering their antigen-binding ability. Thus, for example, the particles may be coated with sheep-anti mouse antibody (SAM) which binds to the Fc portions of IgG mouse antibodies or Protein A which reacts universally with the Fc portions of virtually all IgG antibodies, this bond being cleaved by treatment at relatively low pH, eg pH 2, for a short time, e.g. about 60 seconds. Alternatively, the antibody may be covalently bonded to an antigen and the magnetic particles may carry an antibody forming a weak bond with that antigen; such a bond may be cleaved by treatment with an excess of the antigen (see UK Patent 2012954 of Baxter Travenol Labs Inc). Other reversible bonds include disulphide bonds between an SH group on the antibody and an SH group on the magnetic particles, (which disulphide bond may be cleaved reductively under gentle conditions), ester bonds between a carbonyl group on the magnetic particles and a hydroxyl group on the antibody, which may be cleaved by treatment with an appropriate esterase, and peptide bonds which may be cleaved by a proteolytic enzyme such as chymopapain (C.I. Civin et al, in press).

The above-described methods, while permitting cleavage of the hand between the cell-binding antibody and the magnetic particle, do have the result however that at least a portion of the cells liberated from the particles will have the binding antibody still attached; antigen/antibody binding is often difficult to reverse without destructive effects.

Whilst this is not relevant in the negative cell selection step where the selected cells are discarded, it is generally preferable, to ensure that in the positive cell selection step the cells are liberated without any part of the binding antibody remaining attached. This is particularly so where the cells are intended for transplantation, since any foreign substance remaining attached to the surface of liberated cells will tend to interfere with cell reproduction and viability; transplanted cells are intended to colonise depleted bone marrow and hence it is particularly important that their reproductive potential be undiminished.

In WO91/15766 we describe a particularly advantageous method for detaching antibody-bound cells from particles whereby the linkage between the cell surface antigen and antibody bound to the particle is broken under mild conditions and avoiding destructive effects by reacting with a further antibody, or a binding activity-retaining fragment thereof, (e.g. an F(ab)₂, Fab or Fv fragment) directed against the anti-cell antibody. Thus, since in this method the cell-binding activity remains attached to the particle, and the binding between the cell antigen and the antibody is simply reversed there is no problem with unwanted parts of the antibody remaining bound to the cell. Such a cell liberation method is particularly preferred in the positive cell selection step of the present invention. The further anti-antibody is preferably directed against the Fab region of the cell-binding antibody.

The initial population of haemopoietic cells containing the desired HPC will commonly be derived from bone marrow but may also be obtained from foetal and umbilical cord blood or even adult human blood. In each case, mononuclear cells may be isolated initially, for example by centrifugation using a density gradient.

The magnetic particles used in the above process are preferably superparamagnetic, to avoid permanent magnetisation and hence clumping, and are advantageously monodisperse to provide uniform reaction kinetics and magnetic separation. The superparamagnetic, monodisperse particles according to EP106873 sold by Dynal A.S, Oslo, Norway, are particularly suitable. Such particles can carry functional groups such as hydroxyl, tosyl, carboxyl or amino groups which can be used to bond to a suitable ligand for reversible attachment of the antibody. Using such particles we have found that for optional cell separation 30 to 70 e.g. about 50, magnetic particles may be used.

The negative selection process of step (1) may be repeated in order to maximise the removal of cells in this stage. Two cycles of treatment with magnetic beads carrying antibody AB-4 have produced a 10⁶ fold reduction in lymphoma contamination of a bone marrow preparation.

The greatly enriched stem cell population produced by the process of the invention may be used directly as an autologous bone marrow transplant, particularly in the treatment of a subgroup of patients with non-Hodgkin's lymphomas. However, since very few cells will remain after the enrichment procedure, it may be advantageous to multiply these in a long time marrow culture (Andrews, J. Expl. Med. 169, 1721-31, 1989) before transplantation. The selected stem cells may be simply injected intravenously into the patient since they have been shown to target the bone-marrow growth sites where they can proliferate to replace bone marrow killed by cytotoxic treatment or irradiation.

The pluripotent HPC are capable of self renewal to a greater extent than more differentiated cells and are thus the most important component in the selected population of HPC. Furthermore, because so few antigens are expressed on the cell surface at this stage, they are often termed 'naive' in the sense that they are not recognised by the host immune system. For this reason they can also possibly be used in transplantation into other hosts without substantial risk of host-graft rejection.

According to a preferred feature of the invention we provide a population of pluripotent haemopoietic progenitor cells characterised in that substantially all the cells carry the antigen CD-34 but lack the DP or DQ antigens of MHC Class II. Such cells are preferably unattached to (i.e. detached from) magnetic particles, in order that they may be used directly in bone marrow replacement.

The various reactants required to perform the method of the invention may conveniently be supplied in a kit form. Thus in a further aspect there is provided a kit comprising
(i) a negative selection antibody reactive with DP, DQ or DR antigens of the MHC Class II but not the monomorphic epitope of the DR antigen on HPC, said antibody being optionally bound to magnetic particles;
(ii) a positive selection antibody reactive with an antigen on HPC, said antibody optionally being bound to magnetic particles;
(iii) where the antibody of (i) and (ii) is not bound to magnetic particles, magnetic particles capable of binding with one or both of said antibodies without removing the antigen-binding ability thereof; and, optionally,
(iv) an antibody which reacts with the positive selection antibody (ii) whereby binding between the latter and a cell antigen is reversed.

The following examples are given by way of illustration only:

### EXAMPLE 1

Bone marrow aspirates were initially centrifuged on a density gradient (Isopaque-Ficoll). The mononuclear cell fraction was collected. Monoclonal antibody AB4 was bonded to M-450 Dynabeads (Dynal AS, Oslo, Norway) as described in Cancer Research, 47, 846-851, 1987).

The mononuclear cell fraction above was dispersed in RPMI 1640 medium containing 10% fetal calf serum at 10⁷ cells/ml and incubated with the antibody-treated Dynabeads for 30 minutes at 4°C with occasional rotation. A magnet was applied to the wall of the vessel to aggregate the Dynabeads and the supernatant was transferred to another vessel.

The above supernatant was treated with Dynabeads carrying monoclonal antibody HKB1 (which is pan MHC Class II specific) for 30 minutes at 4°C and the rosetted cells magnetically aggregated to allow removal of the supernatant. The cells were washed by resuspension in growth medium and this washing procedure repeated 3 more times.

The Dynabeads and attached cells were transferred to a glycine buffer (pH2; 0.1M NaCl, 1mMCaCl₂; 0.5mm MgCl₂; 5mM KCl, 52 mM glycine, 1mg/ml D-glucose) at 4°C for 60 seconds and then the medium was neutralised to pH 7.0 with bicarbonate buffer. The liberated Dynabeads were removed magnetically to leave an enriched preparation of stem cells.

### EXAMPLE 2

Bone marrow mononuclear cells are incubated in growth medium (RMPI 1640) with immunomagnetic beads (Dynabeads) coated with the anti-CD34 antibody B1-3C5, at a ratio of beads: total cells of 1: 1, and rosetted for 45 min. at 4°C with gentle rotation and the beads removed with a magnet. After washing (x7 in growth medium) the beads are detached from the CD34 cells by adding anti-(anti-CD34) (1 Unit DETACHaBEAD; Dynal AS Oslo) per 10⁶ cells and rotating at room temperature for 45 min.

A typical detachment will contain 2 x 10⁶ rosettes in 300 »l growth medium + 5% fecal calf serum (FCS). The detachment is close to 100% efficient. The viability ≧ 95% as measured by viability counting.

The isolated CD34⁺ cell population then undergoes negative depletion by applying two rounds of rosetting with immunomagnetic beads coated with antibodies reactive with subpopulations of the CD34⁺ cells. Positively selected CD34⁺ cells are incubated with Dynabeads carrying monoclonal antibody AB-4 in a concentration of 10⁷ cells per ml in growth medium 5% FCS (for reference see Cancer Res. 47-846-855 1987) in a ratio of 50 beads per cell and incubated as above. Rosetted cells are removed by a magnet. This step is repeated once by adding further AB-4 beads and magnetic removal.

The population of cells (suspended in growth medium) remaining after removal of AB-4 positive cells was added to confluent layers of stromal cells in petri dishes prepared by the method of Gordon et al (J. Cell. Phys 130; 150-156, 1987). After incubation at 37°C in 5% humidified CO₂ in air for 2 hours, the stromal layers were washed to remove any unattached cells, and incubated for a further 14 days. At the end of the incubation period, blast colonies were seen to have formed, adhering to the stromal cells. The selected cells were thus shown to be colony forming cells (BL-CFC).

## Claims

1. A method of separating haemopoietic progenitor cells (HPC) from within a mixed population of haemopoietic cells, which may contain malignant cells, characterised in that (1) said mixed population is treated with one or more negative selection antibodies reactive with the DP, DQ or DR antigens of the MHC Class II but not with the monomorphic epitope of the DR antigen on the HPC, said negative selection antibody(s) being bound to magnetic particles before or after binding to said cells and the magnetic particles and attached negative selection antibody and cells being removed to leave a negatively selected population of cells including at least HPC, and (2) HPC and any cells associated therewith are treated with a positive selection antibody reactive with an antigen on said HPC, said antibody being bound to magnetic particles before or after binding to cells carrying said antigen, the magnetic particles and attached cells being separated from other cells by magnetic aggregation and the cells optionally being liberated from the magnetic particles to leave a positively selected population of cells containing said HPC, steps (1) and (2) being effected in either order so that the said positively selected population of cells is the mixed population treated in step (1) or the said negatively selected population of cells are the cells treated in step (2).

2. A method as claimed in claim 1 for separating pluripotent HPC wherein said positive selection antibody is reactive with an antigen expressed on HPC at the pluripotent stage.

3. A method as claimed in claim 1 or claim 2, wherein said negative selection antibody is reactive with DR antigens of the MHC Class II other than the monomorphic epitope of the DR antigen on haemopoietic HPC.

4. A method as claimed in any one of claims 1 to 3 wherein said negative selection antibody is used in combination with one or more antibodies selected from B-cell lineage specific antibodies, T-cell lineage specific antibodies or antibodies directed against cells of the myeloid lineage.

5. A method as claimed in claim 4 wherein said B-cell lineage specific antibody is AS-1, said T-cell lineage specific antibody is anti-CD2 or anti-CD7 and said myeloid cell directed antibody is anti-CD33.

6. A method as claimed in any one of claims 1 to 5 wherein said positive selection antibody is reactive with the CD34 antigen.

7. A method as claimed in claim 1 wherein said negative and/or said positive selection antibody is of the IgM class.

8. A method as claimed in any of claims 1-7 wherein said cells from the positive cell selection step are liberated from the magnetic particles by the addition of an antibody, or fragment thereof, binding to the positive selection antibody whereby the binding of the positive selection antibody with the cell-antigen is reversed.

9. A kit comprising
(i) a negative selection antibody reactive with DP, DQ DQ or DR antigens of the MHC Class II but not the monomorphic DR antigen on HPC, said antibody being optionally bound to magnetic particles;
(ii) a positive selection antibody reactive with an antigen on HPC, said antibody optionally being bound to magnetic particles;
(iii) where the antibody of (i) and (ii) is not bound to magnetic particles, magnetic particles capable of binding with one or both of said antibodies without removing the antigen-binding ability thereof and, optionally
(iv) an antibody which reacts with the positive selection antibody (ii) whereby binding between the latter and a cell antigen is reversed.

10. A population of haemopoietic progenitor cells for use in therapy, characterised in that substantially all the cells carry the antigen CD-34 but lack the DP or DQ antigens of MHC Class II.

## Patentansprüche

1. Verfahren zur Trennung hämatopoetischer Vorläuferzellen (HPC) aus einer Mischpopulation hämatopoetischer Zellen, die maligne Zellen enthalten kann, dadurch **gekennzeichnet**, daß man (1) die Mischpopulation mit einem oder mehreren Antikörper(n) negativer Selektion, der/die mit den DP-, DQ- oder DR-Antigenen der MHC-Klasse II, aber nicht mit dem monomorphen Epitop des DR-Antigens auf den HPC reaktionsfähig ist/sind, behandelt, wobei man den/die Antikörper negativer Selektion an Magnetteilchen vor oder nach der Bindung an die Zellen bindet, und man die Magnetteilchen und den angeknüpften Antikörper negativer Selektion und die Zellen unter Zurücklassung einer Zellpopulation negativer Selektion einschließlich mindestens HPC entfernt, und (2) man die HPC und alle damit assoziierten Zellen mit einem Antikörper positiver Selektion, der mit einem Antigen auf den HPC reaktionsfähig ist, behandelt, wobei man den Antikörper an Magnetteilchen vor oder nach der Bindung an Zellen, die dieses Antigen tragen, bindet, wobei man die Magnetteilchen und die angeknüpften Zellen von den anderen Zellen durch magnetische Aggregation trennt und die Zellen gegebenenfalls aus den Magnetteilchen unter Zurücklassung einer Zellpopulation positiver Selektion, die die HPC enthält, freisetzt, wobei man die Stufen (1) und (2) in beliebiger Reihenfolge durchführt, so daß die Zellpopulation positiver Selektion die in Stufe (1) behandelte Mischpopulation ist, oder die Zellpopulation negativer Selektion die in Stufe (2) behandelten Zellen ist.

2. Verfahren nach Anspruch 1 zur Trennung pluripotenter HPC, wobei der Antikörper positiver Selektion mit einem auf den HPC im pluripotenten Stadium exprimierten Antigen reaktionsfähig ist.

3. Verfahren nach Anspruch 1 oder 2, wobei der Antikörper negativer Selektion mit DR-Antigenen der MHC-Klasse II, ausgenommen das monomorphe Epitop des DR-Antigens auf den hämatopoetischen HPC, reaktionsfähig ist.

4. Verfahren nach einem der Ansprüche 1 bis 3, wobei man den Antikörper negativer Selektion in Kombination mit einem oder mehreren Antikörpern, ausgewählt aus B-zellinienspezifischen Antikörpern, T-zellinienspezifischen Antikörpern oder Antikörpern gegen Zellen der myeloiden Linie, verwendet.

5. Verfahren nach Anspruch 4, wobei der B-Zellinienspezifische Antikörper AB-1, der T-zellinienspezifische Antikörper Anti-CD2 oder Anti-CD7, ist und der gegen die Myeloidzellen gerichtete Antikörper Anti-CD33 ist.

6. Verfahren nach einem der Ansprüche 1 bis 5, wobei der Antikörper positiver Selektion mit dem CD34-Antigen reaktionsfähig ist.

7. Verfahren nach Anspruch 1, wobei der Antikörper negativer und/oder positiver Selektion der IgM-Klasse angehört.

8. Verfahren nach einem der Ansprüche 1 bis 7, wobei man die Zellen aus der Stufe der positiven Zellselektion aus den Magnetteilchen durch Zugabe eines Antikörpers oder Fragments davon, Binden an den Antikörper positiver Selektion freisetzt, wodurch die Bindung des Antikörpers positiver Selektion mit dem Zellantigen aufgehoben wird.

9. Kit, umfassend
(i) einen Antikörper negativer Selektion, der mit DP-, DQ- oder DR-Antigenen der MHC-Klasse II reaktionsfähig ist, ausgenommen das monomorphe DR-Antigen auf HPC, wobei der Antikörper gegebenenfalls an Magnetteilchen gebunden ist;
(ii) einen Antikörper positiver Selektion, der mit einem Antigen auf HPC reaktionsfähig ist, wobei der Antikörper gegebenenfalls an Magnetteilchen gebunden ist;
(iii) sofern der Antikörper von (i) und (ii) nicht an Magnetteilchen gebunden ist, Magnetteilchen mit der Fähigkeit, einen oder beide Antikörper zu binden, ohne die antigenbindende Fähigkeit davon zu beseitigen, und gegebenenfalls
(iv) einen Antikörper der mit dem Antikörper der positiven Selektion (ii) reagiert, wodurch die Bindung zwischen dem zuletztgenannten und dem Zellantigen aufgehoben wird.

10. Population hämatopoetischer Vorläuferzellen zur therapeutischen Verwendung, dadurch **gekennzeichnet**, daß im wesentlichen alle Zellen das CD-34-Antigen tragen, ihnen aber die DP- oder DQ-Antigene der MHC-Klasse II fehlen.

## Revendications

1. Procédé de séparation de cellules progénitrices hématopoïétiques (HPC) dans une population mixte de cellules hématopoïétiques, qui peut contenir des cellules malignes, caractérisé en ce que (1) on traite ladite population mixte par un ou plusieurs anticorps de sélection négative, réactifs avec les antigènes DP, DQ ou DR de la classe MHC II, mais non avec l'épitope monomorphe de l'antigène DR sur les HPC, le ou lesdits anticorps de sélection négative étant liés à des particules magnétiques avant ou après la liaison auxdites cellules et les particules magnétiques et l'anticorps de sélection négative attaché et les cellules étant éliminés pour laisser subsister une population négativement sélectionnée de cellules comprenant au moins des HPC et (2) les HPC et toutes cellules qui y sont associées sont traitées par un anticorps de sélection positive, réactif avec un antigène sur lesdits HPC, ledit anticorps étant lié à des particules magnétiques avant ou après la liaison à des cellules portant ledit antigène, les particules magnétiques et les cellules attachées étant séparées d'autres cellules par agrégation magnétique et les cellules éventuellement libérées à partir des particules magnétiques, pour laisser subsister une population positivement sélectionnée de cellules contenant lesdites HPC, les étapes (1) et (2) étant effectuées en n'importe quel ordre, si bien que ladite population positivement sélectionnée de cellules est la population mixte traitée au cours de l'étape (1), ou bien ladite population négativement sélectionnée de cellules constitue les cellules traitées au cours de l'étape (2).

2. Procédé suivant la revendication 1 de séparation d'HPC pluripotentes, caractérisé en ce que l'anticorps de sélection positive précité réagit avec un antigène exprimé sur les HPC à l'état ou stade pluripotent.

3. Procédé suivant la revendication 1 ou la revendication 2, caractérisé en ce que l'anticorps de sélection négative précité est réactif avec des antigènes DR de la classe MHC II autres que l'épitope monomorphe de l'antigène DR sur les HPC hématopoïétiques.

4. Procédé suivant l'une quelconque des revendications 1 à 3, caractérisé en ce que ledit anticorps de sélection négative est utilisé en combinaison avec un ou plusieurs anticorps choisis parmi des anticorps spécifiques de lignée de cellules B, des anticorps spécifiques de lignée de cellules T, ou des anticorps dirigés contre des cellules de la lignée myéloïde.

5. Procédé suivant la revendication 4, caractérisé en ce que ledit anticorps spécifique de la lignée des cellules B est l'AB-1, ledit anticorps spécifique de la lignée des cellules T est l'anti-CD2 ou l'anti-CD7 et ledit anticorps dirigé contre des cellules de la lignée myéloïdes est l'anti-CD33.

6. Procédé suivant l'une quelconque des revendications 1 à 5, caractérisé en ce que ledit anticorps de sélection positive est réactif avec l'antigène CD34.

7. Procédé suivant la revendication 1, caractérisé en ce que ledit anticorps de sélection négative et/ou de sélection positive appartient à la classe IgM.

8. Procédé suivant l'une quelconque des revendications 1 à 7, caractérisé en ce que lesdites cellules de l'étape de sélection de cellules positive sont libérées des particules magnétiques par l'addition d'un anticorps, ou d'un fragment de celui-ci, se liant à l'anticorps de sélection positive, si bien que la liaison de l'anticorps de sélection positive avec l'antigène cellulaire est inversée.

9. Trousse comprenant
(i) un anticorps de sélection négative, réactif avec des antigènes DP, DQ ou DR de la classe MHC II, mais non avec l'antigène DR monomorphe sur les HPC, ledit anticorps étant facultativement lié à des particules magnétiques,
(ii) un anticorps de sélection positive, réactif avec un antigène sur les HPC, ledit anticorps étant facultativement lié à des particules magnétiques,
(iii) lorsque l'anticorps de (i) et de (ii) n'est pas lié à des particules magnétiques, des particules magnétiques capables de se lier à l'un des ou au deux anticorps précités, sans enlever l'aptitude de liaison à l'antigène desdits anticorps et, facultativement,
(iv) un anticorps qui réagit avec l'anticorps de sélection positive (ii) si bien que la liaison entre ce dernier et un antigène cellulaire est inversée.

10. Population de cellules progénétrices hématopoïétiques pour l'utilisation dans le domaine thérapeutique, caractérisée en ce que sensiblement toutes les cellules portent l'antigène CD-34 mais ne comportent pas des antigènes DP ou DQ de la classe MHC II.
